(19) Europäisches Patentamt
European Patent Office
Office européen des brevets

(11) Publication number: **0 215 639 B1**

(12) **EUROPEAN PATENT SPECIFICATION**

(45) Date of publication of patent specification: **29.08.90**

(51) Int. Cl.[5]: **C 07 C 303/34,** C 07 C 257/14, C 07 D 277/48

(21) Application number: **86306988.6**

(22) Date of filing: **10.09.86**

(54) **Propionamidine derivatives.**

(30) Priority: **11.09.85 HU 342385**

(43) Date of publication of application: **25.03.87 Bulletin 87/13**

(45) Publication of the grant of the patent: **29.08.90 Bulletin 90/35**

(84) Designated Contracting States: **CH DE FR GB IT LI NL SE**

(56) References cited:

**CHEMICAL ABSTRACTS, vol. 107, no.5, 3rd August 1987, page 701, abstract no. 39796s, Columbus, Ohio, US; & ES-A-526 303 (INKE S.A.) 01-04-1985**

(73) Proprietor: **RICHTER GEDEON VEGYESZETI GYAR R.T.**
**Gyömröi ut 19-21**
**H-1475 Budapest X (HU)**

(72) Inventor: **Bod, Peter**
**Wekerle u.46**
**H-2230 Gyomro (HU)**
Inventor: **Harsanyi, Kalman**
**Fodor u. 12**
**H-1126 Budapest (HU)**
Inventor: **Csongor, Eva**
**Vermezo u. 8**
**H-1012 Budapest (HU)**
Inventor: **Bogsch, Erik**
**Liderc u. 40-42**
**H-1121 Budapest (HU)**
Inventor: **Fekecs, Eva**
**Allomas u. 13**
**H-1102 Budapest (HU)**
Inventor: **Trischler, Ferenc**
**Uttoro u. 16**
**H-1171 Budapest (HU)**
Inventor: **Domany, Gyorgy**
**Bimbo u. 114/a**
**H-1022 Budapest (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Inventor: Szabadkai, Istvan
Sallai I. u. 5/a
H-1136 Budapest (HU)
Inventor: Hegedus, Bela
Bartok Bela u. 82
H-1113 Budapest (HU)

(74) Representative: Pett, Christopher Phineas et al
Frank B. Dehn & Co. European Patent Attorneys
Imperial House 15-19 Kingsway
London WC2B 6UZ (GB)

**Description**

The invention relates to new propionamidine derivatives, to processes for their preparation and to their use in the synthesis of an anti-ulcer agent.

Famotidine [N-sulfamyl-3-(2-guanidino-thiazol-4-yl-methylthio)-propionamidine] is an anti-ulcer agent providing excellent results in the treatment of gastric and intestinal ulceration.

In for example U.S. patent specification No. 4,496,737 there is described an N-sulfamylacrylamidine compound of formula (IV)

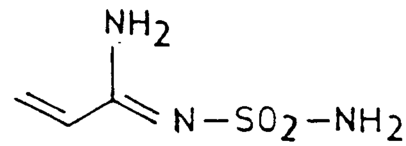

(IV)

As described therein this compound is prepared starting from 3-methylthio-propionitrile by a long and cumbersome four-step synthesis, with a total yield of 5.5%. In addition to the extremely low yield by which it can be prepared a further disadvantage of this compound is that its purification is very difficult even by chromatography. Accordingly, its use in the preparation of famotidine does not make possible an industrially applicable, economic synthesis. It would be desirable, therefore, to provide new intermediates from which famotidine can be prepared economically.

We have found that N-sulfamyl-3-halopropionamide derivatives of the formula (I),

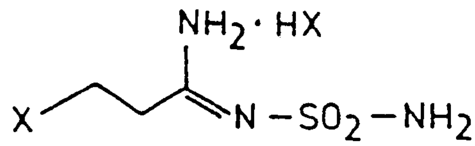

(I)

(wherein X is halogen) are not only easy to prepare but can also easily be converted into famotidine. These compounds thus form a feature of the present invention.

According to a further feature of the invention the new propionamidine derivatives of the formula (I) may be prepared by reacting a 3-halopropionitrile of the formula (III)

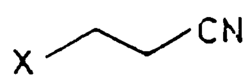

(III)

(wherein X is as defined above), with sulfamide of the formula (II)

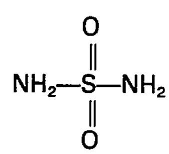

(II)

in the presence of a hydrogen halide.

According to a preferred embodiment of the invention the 3-halopropionitrile is prepared *in situ* from acrylonitrile by hydrogen halide addition (e.g. by reaction with gaseous hydrogen chloride or hydrogen bromide).

Thus according to a particularly preferred embodiment of the invention the new compounds of formula (I) can for example be prepared by introducing dry hydrogen chloride gas into a stirred solution of the sulfamide in an excess of 3-chloropropionitrile (or acrylonitrile). The precipitated crystalline compound of formula (I) may then be filtered off upon cooling and optionally after dilution with an organic solvent, washed with acetone and dried.

Favourable results are obtained by reacting 1 mole of sulfamide with 1.5 to 8 moles, preferably 1.8 to 2.5 moles, of the 3-halopropionitrile derivative of the formula (III), at 10°C to 100°C, preferably 10°C to 60°C, for 1 to 100, preferably 20 to 60 hours, while a hydrogen halide is passed through the solution. Solvents, such as e.g. ethers, may be used.

The main advantages of the process according to the invention are as follows:

a) due to the direct sulfamide nitrile addition the isolation and reaction of the iminoether base intermediate, which is liable to decomposition, can be avoided as can the purification problems associated therewith;

b) the reactant, that is sulfamide and either 3-chloropropionitrile or acrylonitrile and hydrogen chloride gas are readily accessible, cheap industrial raw materials;

c) the product is not hygroscopic, well crystallizable, is easy to filter and is obtained with a yield of at least 70%.

The N-sulfamyl-3-halopropionamidine salts according to the invention can readily be converted into famotidine and with an excellent yield. More particularly, famotidine is prepared starting from the new intermediates according to the invention by S-alkylating 2-guanidino-thiazol-4-yl-methyl-methanethiol, obtainable from S-(2-guanidino-thiazol-4-yl-methyl)-isothiourea dihydrochloride by *in situ* treatment with a base, with an N-sulfamyl-3-halopropionamidine hydrohalide of the formula (I), wherein X is as described above. The yields obtained by this process are high (around 70%).

A further advantage of the process according to the invention is that the halopropionitrile used in an excess amount or formed can be easily recovered and recycled into the process.

Further details of the invention are illustrated by the aid of the following non-limiting Examples.

Example 1

N-Sulfamyl-3-chloropropionamidine hydrochloride

To a suspension of 9.61 g (0.10 mole) of sulfamide in 60 ml (68 g, 0.76 mole) of 3-chloropropionitrile dry hydrogen chloride gas is introduced under stirring at 50 to 60°C for 5 hours. The gain in weight is about 9 to 12 g. The reaction mixture is then cooled with ice water for one hour and the product is filtered off, washed twice with acetone and dried up to steady weight.

Yield: 14.9 g (67.1%)

Melting point: (142)—144—146°C (decomp).

Analysis:

Calculated: C 16.24%, H 4.08%, Cl 31.90%, N 18.92%;

Found: C 16.30%, H 4.13%, Cl 31.86%, N 18.90%.

Purity: 97.0% (determined by potentiometric titration in a methanolic medium)

IR spectrum (in KBr tablet, determined on a "Perkin-Elmer 257" equipment):

| | |
|---|---|
| C=N | 1675 $cm^{-1}$ |
| $SO_2$ | 1170 $cm^{-1}$ br |
| C—Cl | 660 $cm^{-1}$ |

Proton NMR spectrum ($d_6$-DMSO/$D_2O$, determined on a "Varian EM 260" equipment):

| | |
|---|---|
| =C—$CH_2$— | 3.05 ppm t |
| Cl—$CH_2$— | 4.00 ppm t |
| X—H | 8.50 ppm s(b)* |

Example 2

N-Sulfamyl-3-chloropropionamidine hydrochloride

To a stirred suspension of 9.61 g (0.10 mole) of sulfamide in 30 ml (34 g, 0.38 mole) of 3-chloropropionitrile hydrogen chloride gas is introduced, as described in Example 1. After cooling with ice water for one hour the reaction mixture is diluted with 30 ml of dry diethyl ether and the product is filtered off, washed twice with acetone and dried.

Yield: 16.15 g (72.7%)

Melting point: (142)—144—146°C (decomp).

Example 3

N-Sulfamyl-3-chloropropionamidine hydrochloride

18 to 20 g of hydrogen chloride gas are absorbed in a stirred suspension of 9.61 g sulfamide in 27 g (34 ml, 0.5 mole) of acrylonitrile at 0 to +2°C. The reaction mixture is then heated up to 50°C and hydrogen chloride gas is passed through it for three hours, while the internal temperature is kept between 50°C and 60°C. The thick, white crystal suspension obtained is cooled to room temperature, diluted with 25 ml of diisopropyl ether, cooled with ice water, filtered, washed with acetone and dried.

Yield: 16.0 g (72.0%)

Melting point: (143)—145—147°C (decomp).

Example 4

N-Sulfamyl-3-chloropropionamidine hydrochloride

A suspension of 9.61 g of sulfamide with 26.9 g (24.0 ml, 0.30 mole) of regenerated 3-chloropropionitrile and 10.7 g (23.5 ml, 0.2 mole) of acrylonitrile is stirred continuously whereupon 7 to 8 g of hydrogen chloride gas are absorbed in the suspension at a temperature of 0 to +2°C. The mixture is then heated up to 50°C and hydrogen chloride gas is passed through it for further 3 hours, at a temperature of 50° to 60°C. After dilution with 25 ml of diisopropyl ether and cooling with ice the product obtained is filtered off, washed with acetone and dried.

Yield: 15.5 g (69.8%)

Melting point: (143)—144—146°C (decomp).

Example 5

To a solution of 3.04 g (0.01 mole) of S-(2-guanidino-thiazol-4-yl-methyl)-isothiourea dihydrochloride

and 2.22 g (0.01 mole) of N-sulfamyl-(3-chloro)-propionamidine hydrochloride in 8.0 ml of deionized water a mixture of 4.0 ml of a 10 N sodium hydroxide solution (0.04 mole) and 6.0 ml ethanol is added dropwise, at 25 to 30°C, under stirring. The homogeneous mixture obtained (pH 11), is stirred for a further one and a half hours and cooled with ice water for one hour. The product is filtered off, washed twice with deionized water and twice with isopropanol and dried until steady weight.

2.40 g (71.2%) of famotidine are obtained.

Melting point: (159)—160 to 162°C, decomposition at 165°C

IR spectrum (KBr): $NH_2$ 3506, 3452, 3400;
NH 3360, 3377, 3240;
C=N 1639,
C=N (conj.) 1604 br;
$SO_2$ 1288, 1145 $cm^{-1}$

Proton NMR spectrum (DMSO $d_6$):

| | |
|---|---|
| S— $CH_2$— $CH_2$— N | 2.6 ppm multiplet |
| Ar— $CH_2$— S | 3.6 ppm singlet |
| Ar— H | 6.5 ppm singlet |
| NH, $NH_2$ | 3.5; 6.8; 7.4; 8.3 ppm, br, exchangeable. |

**Claims**

1. Propionamidine derivatives of the formula (I),

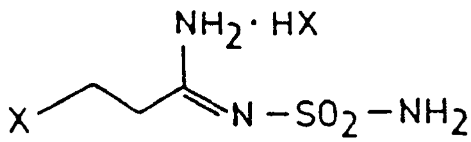

(I)

wherein X is halogen.

2. A compound of formula (I) according to claim 1 which is N-sulfamyl-3-chloropropionamidine hydrochloride.

3. A process for the preparation of propionamidine derivatives as defined in claim 1 which comprises reacting a 3-halopropionitrile of formula (III)

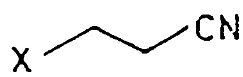

(III)

(wherein X is as defined in claim 1) with sulfamide of the formula (II)

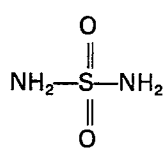

(II)

in the presence of a hydrogen halide.

4. A modification of the process according to claim 3 in which the 3-halopropionitrile of formula (III) is prepared *in situ* from acrylonitrile, by hydrogen halide addition.

5. A process according to claim 4 wherein excess 3-halopropionitrile is recycled.

6. A process according to any one of claims 3 to 5 in which 1.5 to 8 moles of the 3-halopropionitrile are used per mole of sulfamide, the reaction being effected at a temperature of 10 to 100°C, for 1 to 100 hours, under hydrogen chloride gas.

7. A process according to claim 6 in which 1.8 to 2.5 moles of the 3-halopropionitrile are used per mole of sulfamide, the reaction being effect at a temperature of 10 to 60°C for 20 to 60 hours.

8. A process for the preparation of famotidine which comprises S-alkylating 2-guanidino-thiazol-4-yl-methanethiol, obtainable from S-(2-guanidinothiazol-4-yl-methyl)-isothiourea dihydrochloride by *in situ* treatment with a base, with a propionamidine derivative of formula (I) as defined in claim 1.

**Patentansprüche**

1. Propionamidinderivate der Formel (I)

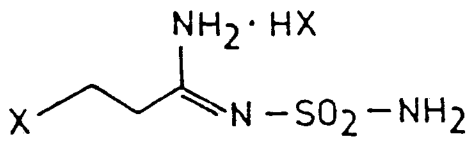

(I)

worin X Halogen ist.

2. Verbindung der Formel I nach Anspruch 1, die N-Sulfamyl-3-chlorpropionamidin-hydrochlorid darstellt.

3. Verfahren zur Herstellung von Propionamidinderivaten nach Anspruch 1, das die Umsetzung eines 3-Halopropionitrils der Formel (III)

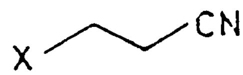

(III)

worin X die in Anspruch 1 angeführte Bedeutung hat, mit einem Sulfamid der Formel (II)

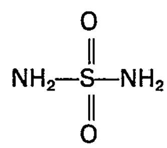

(II)

in Anwesenheit eines Wasserstoffhalogenids darstellt.

4. Modifizierung des Verfahrens nach Anspruch 3, bei dem das 3-Halopropionitril der Formel (III) *in situ* aus Acrylnitril durch Wasserstoffhalogenid-Addition hergestellt wird.

5. Verfahren nach Anspruch 4, worin das überschüssige 3-Halopropionitril zurückgeführt wird.

6. Verfahren nach einem der Ansprüche 3 bis 5, bei dem 1,5 bis 8 mol 3-Halopropionitril pro Mol Sulfamid verwendet werden und die Umsetzung bei einer Temperatur von 10 bis 100°C während 1 bis 100 Stunden unter Chlorwasserstoffgas durchgeführt wird.

7. Verfahren nach Anspruch 6, bei dem 1,8 bis 2,5 Mol 3-Halopropionitril pro Mol Sulfamid verwendet werden und die Umsetzung bei einer Temperatur von 10 bis 60°C während 20 bis 60 Stunden durchgeführt wird.

8. Verfahren zur Herstellung von Famotidin, das die S-Alkylierung von 2-Guanidino-thiazol-4-yl-methanthiol, das aus S-(2-Guanidino-thiazol-4-yl-methyl)-isothioharnstoffdihydrochlorid durch *in situ*-Behandlung mit einer Base hergestellt werden kann, mit einem Propionamidinderivat der Formel (I) nach Anspruch 1, umfaßt.

**Revendications**

1. Dérivés de propionamidine de formule (I),

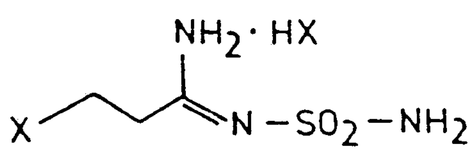

(I)

dans laquelle X est un atome d'halogène.

2. Composé de formule (I) suivant la revendication 1, caractérisé en ce qu'il s'agit du chlorhydrate de N-sulfamyl-3-chloropropionamidine.

3. Procédé pour la préparation de dérivés de propionamidine suivant la revendication 1, caractérisé en ce qu'il comprend la réaction d'un 3-halopropionitrile de formule (III)

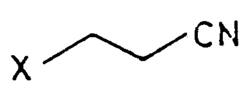

(III)

(dans laquelle X est tel que défini dans la revendication 1) avec un sulfamide de formule (II)

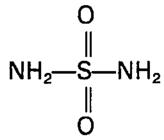

(II)

en présence d'un halogénure d'hydrogène.

4. Modification du procédé suivant la revendication 3, caractérisé en ce que le 3-halopropionitrile de formule (III) est préparé in situ à partir d'acrylonitrile, par addition d'halogénure d'hydrogène.

5. Procédé suivant la revendication 4, caractérisé en ce que le 3-halopropionitrile en excès est recyclé.

6. Procédé suivant l'une quelconque des revendications 3 à 5, caractérisé en ce qu'on utilise de 1,5 à 8 moles du 3-halopropionitrile par mole de sulfamide, la réaction étant effectuée à une température de 10 à 100°C, pendant 1 à 100 heures, dans du chlorure d'hydrogène gazeux.

7. Procédé suivant la revendication 6, caractérisé en ce qu'on utilise de 1,8 à 2,5 moles du 3-halopropionitrile par mole de sulfamide, la réaction étant effectuée à une température de 10 à 60°C, pendant 20 à 60 heures.

8. Procédé pour la préparation de famotidine, caractérisé en ce qu'il comprend la S-alkylation de 2-guanidinothiazol-4-yl-méthanethiol, pouvant être obtenu à partir du dichlorhydrate de S-(2-guanidino-thiazol-4-yl-méthyl)-isothiourée par traitement in situ avec une base, avec un dérivé de propionamidine de formule (I) telle que définie dans la revendication 1.